# EUROPEAN PATENT SPECIFICATION

(11) **EP 4 599 022 B1**
(45) Date of publication and mention of the grant of the patent: **02.12.2026**
(21) Application number: 23783890.9
(22) Date of filing: 05.10.2023
(51) Int. Cl.: C10B 53/07, C08J 11/12, C10G 1/00, C10G 1/10, C10K 1/04, C10K 1/06, C07C 4/22

(54) **METHOD FOR PRODUCING A CRUDE STYRENE OIL MIXTURE RICH IN STYRENE AND STYRENE DERIVATIVES**
VERFAHREN ZUR HERSTELLUNG EINES STYROL- UND STYROL-DERIVATENREICHEN ROH-STYROL-ÖLGEMISCHES
PROCÉDÉ DE PRODUCTION D'UN MÉLANGE D'HUILE DE STYRÈNE BRUTE RICHE EN STYRÈNE ET DÉRIVÉS DE STYRÈNE

(30) Priority: 06.10.2022 EP 22200108
(43) Date of publication of application: 13.08.2025
(73) Proprietor: INEOS Styrolution Group GmbH, 60325 Frankfurt (DE)
(72) Inventor: VERSWYVEL, Michiel, 2800 Mechelen (BE); CAVEYE, Stephanie, 9100 Sint-Niklaas (BE); VRIEZE, Jenoff De, 9000 Gent (BE); STEIJVOORT, Ben Van, 2170 Merksem (BE)
(74) Representative: Jacobi, Markus Alexander
(86) International application number: PCT/EP2023/077524
(87) International publication number: WO 2024/074591

(56) References cited:
- WO-A1-2021/053074
- CA-A- 1 238 597
- US-A1- 2011 067 992

## Description

The invention relates to a method for producing a crude styrene oil mixture, rich in styrene and styrene derivatives, by pyrolysis of a polymer waste stream. The method involving a step of rapid phase separation. The method produces a pyrolysis oil (PO) containing vinylaromatic monomers (M) from a polymer (P) comprising repeating units of said monomers (M). The method comprises steps of pyrolysis of the polymer (P), quenching of the pyrolysis reaction with an aqueous solution (A) or water, phase separation and collecting of the organic phase as the pyrolysis oil (PO), wherein the step of quenching and/or the phase separation involve an aqueous solution of phosphate ions (PO₄³⁻).

### Background

Climate change, environmental pollution, population growth and resource dependency trigger the ecological and economic necessity of the transition from a linear to a circular economy. Since the 1990s, intensive efforts to develop improved processes for the recovery of raw materials from recycling of plastic waste were made. These efforts have not yet resulted in large-scale applications, in particular due to unresolved process engineering problems and economic reasons, such as the non-availability of suitable materials. However, the topic of plastic waste as well as a greater ecological awareness and a need for sustainable solutions have led to a growing interest in chemical recycling.

Not all thermoplastic polymers are equally suited for chemical recycling. The thermal decomposition of polyolefins or polyesters results in mixtures of waxes, light oil and gases. The degradation of polyethylene terephthalate (PET) results in organic acids, mainly benzoic acid and terephthalic acid, which are corrosive and may also cause clogging of the reactor (G. Grause et al., Feedstock recycling of waste polymeric material, in: Journal of Material Cycles and Waste Management, 13(4), 2011, 265-282).

In the case of polystyrene and other styrene-containing polymers, it is possible to depolymerize the polymers into the basic components, especially styrenic monomers, which make polystyrene and other styrene monomer-containing polymers excellent choices for chemical recycling. However, the resulting product mixture of a de-polymerization process needs to be purified in order to use the components as raw material for new purposes, such as polymerization processes.

WO 2018/224482 (INEOS Styrolution) describes a method of pyrolytically de-polymerizing a styrene-containing polymer waste plastic. EP-A 2635043, KR-A 2020 0016935 and CN-A 110869428 deal with de-polymerization as well.

The process of WO 2018/224482 comprises the steps: i) decomposing the styrene-containing polymer waste plastic (K) in a suitable reactor by thermal and optionally shearing energy, wherein the waste plastic (K) is sent to a pyrolysis zone of the reactor and pyrolyzed there, and wherein the styrene component of the waste plastic (K) is at least partly decomposed into styrene monomers and oligomers; ii) capturing the gases formed in step i) and condensing low molecular weight products, comprising the styrene monomers formed; iii) separating the condensed low molecular weight constituents, comprising the styrene monomers formed, via fractional distillation; and iv) sending the styrene oligomers formed in step i) to a steam cracker.

WO 2020/178597 (Oxford Sustainable Fuels Limited) describes a process for upgrading the quality of pyrolysis oil derived from plastic, rubber or a combination thereof, comprising the treatment of a pyrolysis oil with an aqueous solution and optionally a hydrocarbon fluid, wherein the process is intended to remove undesired contaminants from the pyrolysis oil or to enrich the pyrolysis oil with desired components.

WO 2021/110751 (INEOS Styrolution) describes thermoplastic compounds containing recycling material with superior quality. The main aspect of the invention relates to a thermoplastic molding composition (T) comprising recycled polymer material (A), containing ABS.

US 11041123 (Agilyx, 2020) describes systems and methods for recycling waste plastics, including a system for recovering styrene monomer from waste polystyrene. The system includes an apparatus to receive a supply of waste polystyrene and to output a densified polystyrene containing melt; a pyrolysis reactor configured to pyrolyze the densified polystyrene containing melt and recycled oligomers, and to output a hydrocarbon gas stream and a solids residue stream; a quenching apparatus configured to condense out oligomers for routing upstream to the pyrolysis reactor, and to discharge an altered hydrocarbon gas stream for further processing; and a condenser configured to receive the altered hydrocarbon gas stream from the quenching apparatus and condense out styrene to form a styrene monomer oil product.

WO 2021/180893 (Indaver) relates to a two-step pyrolysis process for recovering at least styrene monomer and other low molecular weight liquid polystyrene pyrolysis products from a plastic material containing polystyrene.

After a first cracking step, two gaseous phases are separated and the heavier fraction comprising styrene oligomers is then further cracked. This enables the recovery of a higher amount of the low molecular weight liquid polystyrene pyrolysis products, including styrene monomer and other low molecular weight liquid products, such as alpha-methyl-styrene, cumene, ethyl-benzene and toluene.

In such thermal de-polymerization processes, wherein a styrene-containing polymer waste stream is converted to a crude styrene gas stream (CSG) that is rich in styrene and styrene derivatives, a cost- and energy-efficient and simple way of terminating the pyrolysis reaction and condensing the CSG stream into a crude styrene oil or pyrolysis oil (CSO) includes quenching the reaction with water. In continuous de-polymerization processes, typically a continuous stream of CSG is withdrawn from the de-polymerization reactor, which can then be quenched and condensed with water in a separate device. In a typical process, but not limited to this, the CSG is quenched with finely dispersed water droplets (i.e. a spray). The process can run in one single stage or multiple consecutive stages condensing different portions of the CSG into different fractions of the CSO. In at least one of these stages water is used as quenching and condensing medium but also other media, like hydrocarbons, can be used as quenching and condensing media. The quenching with water allows a fast termination and fast cooling of the CSG stream and hence, the formation of by-products downstream of the reactor, when the CSG is still hot, is reduced.

In the collection vessel after this quenching and condensation step, a stable emulsion and dispersion of the condensed CSO in water can occur. This considerably impedes further processing due to slow or completely absent phase separation into a clear aqueous layer and a CSO layer, which would allow the CSO layer to be separated (i.e. decanted). Such emulsions and dispersions can exist and remain stable even for several days. Although phase separation does occur to a certain extent, even immediately after quenching, it is usually only possible to collect a portion of the organic phase directly after quenching, followed by further processing, whereas the rest of the CSO remains dispersed in the aqueous phase. Furthermore, the aqueous phase containing CSO often cannot be recycled and utilized for quenching, since certain components in the CSO may damage or block conduits or other mechanical components used for transferring the aqueous phase back to the quenching device.

Especially on an industrial scale, it is inconvenient and cost-ineffective to wait for the CSO to separate from the aqueous phase, since the processes need to be interrupted eventually, to collect the CSO separating from the aqueous phase. This particularly applies to continuous processes on an industrial scale.

Therefore, in continuous industrial processes, a large amount of the aqueous phase containing substantial amounts of CSO may have to be discarded, which is undesirable in terms of process efficiency and sustainability. Alternatively, other means for terminating the de-polymerization reaction, like classical condensation of the CSG at the cold walls of e.g. a tube-and-shell condenser, have to be employed instead of quenching and condensing with water, which, however decreases the styrene yield in the CSO due to the slow cooling process and resultant increased amount of formed by-products. In addition, this process is less efficient and may increase the complexity and costs of the process e.g. due to the high velocity of the CSG exiting the pyrolysis reactor.

CN 114053759 A (Th-Unis Insight, 2020) refers to a process for dehydrating aqueous organic compounds, wherein dehydration of a water-containing organic mixture is performed by adding salt. In several phase separation steps of the process, a dehydrating agent selected from anhydrous salt, hydrated salt, nearly saturated salt solution, saturated salt solution and/or supersaturated salt solution is added to the aqueous organic matter. The mixture is left to form an upper organic solution and a lower aqueous salt solution. In one step, the water content of the organic matter can be reduced from 30-40 wt.% to less than 10 wt.%.

JP 2018-103159 A (Kobelco Eco Solutions, 2016) relates to a water treatment method and a waste oil treatment method, especially to a method capable of reducing the phosphate ion concentration in an organic phase.

WO 2010/033789 and US 8741258 (University of Massachusetts) describes the processing of bio-oil derived from fast pyrolysis of biomass, which is a complex mixture of various compounds including water and organic solvents and materials. One processing step is an aqueous extraction to separate water-soluble and water-insoluble fractions. Additives may be included in the aqueous phase to facilitate the separation of the water-soluble and insoluble phases, in particular, salts and/or buffering agents may be added to increase the ionic strength of the aqueous phase to facilitate phase separation, and to adjust the pH of the resulting aqueous phase.

WO 2021/053074 discloses a process for the preparation of styrene monomers by de-polymerising polystyrene, and a device for carrying out the process, and to the use of a fluidised bed reactor for the depolymerisation of polystyrene. The process involves using a water mist for condensing the depolymerisation products, and separation of an organic fraction.

A process sufficiently satisfying all needs described above in terms of industrial application, sustainability, economy and efficiency of quenching, condensing and processing aqueous CSO mixtures is still not available.

An objective of the present invention is to provide a cost-efficient, preferably continuous, process for producing a pyrolysis oil (PO) from a pyrolysis gas stream containing vinylaromatic monomers (M) from a polymer (P) comprising repeating units of said monomers (M), wherein the recovery rate of pyrolysis oil (PO) available after termination of the de-polymerization reaction is increased in relation to other processes known in the art. Preferably, the polymer originates from styrene-containing polymer waste. Accordingly, the pyrolysis oil is preferably a crude styrene pyrolysis oil (CSO).

Therefore, a first aspect of the invention is a method for producing a pyrolysis oil (PO) containing vinylaromatic monomers (M), preferably styrene, from a polymer (P) comprising repeating units of said monomers (M), wherein the method comprises the steps:
a. Pyrolysis of the polymer (P) comprising repeating units of vinylaromatic monomers (M), to obtain a vapour (V) containing said monomers (M);
b. Condensing at least a portion of the monomers (M) by contacting the vapour (V) with an aqueous solution (A) or water, to obtain a heterogeneous mixture of an organic phase containing vinylaromatic monomers (M) and an aqueous phase;
c. Performing a phase separation between the organic phase and the aqueous phase; and
d. Collecting the organic phase containing vinylaromatic monomers (M), to obtain the pyrolysis oil (PO) containing vinylaromatic monomers (M),
wherein in step c. the aqueous phase contains at least one phosphate salt in an amount leading to a phosphate (PO₄³⁻) concentration of at least 0.01 mol/L, more preferably at least 0.1 mol/L, more preferably 0.2 to 2 mol/L, more preferably 0.4 to 1 mol/L. Preferably, the method is continuous. The at least one phosphate salt can be introduced into the aqueous solution (A) used in step b. for condensing monomers (M), or is introduced into the heterogeneous mixture between step b. and c., or in step c. after obtaining the heterogeneous mixture in step b., or a combination of these options.

The pyrolysis oil (PO) obtained from the process of the invention typically contains at least 10% by weight, often at least 40% by weight, often at least 50% by weight, in some cases at least 60% by weight, based on the total weight of the pyrolysis oil (PO), of vinylaromatic monomers (M).

The method may further comprise additional steps, wherein the pyrolysis oil (PO) or individual components thereof, such as monomers (M), are purified and/or isolated. Preferably, the method further comprises the step of distillation of the organic phase containing the pyrolysis oil (PO).

Another aspect of the present invention is a system configured for performing the method of the invention, comprising a means for carrying out a pyrolysis of a polymer (P) comprising repeating units of vinylaromatic monomers (M), a means for quenching the pyrolysis reaction with an aqueous solution (A) or water, a means for performing phase separation, a means for collecting the organic phase from the means for performing phase separation, and a means for dosing salt, configured to dose at least one phosphate salt into the means for performing phase separation, and optionally a distillation means.

The use of an aqueous solution containing a phosphate salt in an amount leading to a phosphate (PO₄³⁻) concentration of > 0 mol/L preferably at least 0.01 mol/L, more preferably at least 0.1 mol/L, more preferably 0.2 to 2 mol/L, more preferably 0.4 to 1 mol/L for quenching a pyrolysis reaction for the recovery of vinylaromatic monomers (M) from a polymer (P) comprising repeating units of said vinylaromatic monomers (M), is another aspect of the present invention.

Moreover, an aspect of the present invention is a method for increasing the speed of phase separation of a heterogeneous liquid mixture comprising vinylaromatic monomers (M) obtained from pyrolysis of a polymer (P), and an aqueous solution (A) or water, wherein the process comprises the step of adjusting the amount of phosphate ions in the aqueous phase of the heterogeneous mixture to a concentration of at least 0.01 mol/L, preferably of at least 0.1 mol/L, more preferably 0.2 to 2 mol/L, more preferably 0.4 to 1 mol/L.

Yet another aspect of the present invention is a heterogeneous liquid mixture comprising an organic phase comprising vinylaromatic monomers (M) obtained from pyrolysis of a polymer (P), and an aqueous phase comprising at least one phosphate salt in an amount leading to a phosphate (PO₄³⁻) concentration of at least 0.01 mol/L, preferably of at least 0.1 mol/L, more preferably 0.2 to 2 mol/L, more preferably 0.4 to 1 mol/L.

Another aspect of the invention is the use of an aqueous solution (A) containing a phosphate salt in an amount leading to a phosphate (PO₄³⁻) concentration of > 0 mol/L preferably at least 0.01 mol/L, more preferably at least 0.1 mol/L, more preferably 0.2 to 2 mol/L, more preferably 0.4 to 1 mol/L, for washing a pyrolysis oil (PO) containing vinylaromatic monomers (M) obtained from pyrolysis of a polymer (P) comprising repeating units of said monomers (M).

The invention and the components used therein are discussed below in more detail.

The terms "pyrolysis" and "depolymerization", in the present context are understood as synonyms, and refer to the at least partial decomposition, preferably thermal decomposition, optionally in the presence of catalysts, of a polymer, yielding the monomers from which it was prepared.

The vinylaromatic monomers (M) may be any vinylaromatic monomers, such as styrene, alpha-methyl styrene, and other substituted styrenes. Preferably, the vinylaromatic monomers (M) comprise, more preferably consist of, styrene. The terms "monomers (M)" and "vinylaromatic monomers (M)" are to be understood as synonyms, as long as "(M)" is present.

The polymer (P), from which the monomers (M) are obtained, may be any polymer comprising repeating units of said monomers (M), which can be subjected to pyrolysis, in order to obtain the monomers (M). The term "polymer" includes both homopolymers of monomers (M) or copolymers of monomers (M) with one or more other monomers. Preferably, the polymer (P) originates from polymer waste, more preferably post-consumer polymer waste.

### Step a.

Step a. of the inventive method comprises the pyrolysis of the polymer (P) comprising repeating units of vinylaromatic monomers (M), preferably styrene. The polymer (P) as such may be subjected to pyrolysis, or it may be subjected to pyrolysis as part of a composition. For example, if polymer (P) originates from polymer waste, preferably post-consumer polymer waste, it may be isolated from the polymer waste before pyrolysis, or the polymer waste as such may be subjected to pyrolysis. Preferably, the polymer (P) or the composition (preferably polymer waste) comprising polymer (P) is provided as a continuous stream into the pyrolysis reactor.

The composition comprising polymer (P) may further comprise other components, e.g. other polymeric materials, free monomers (M), oligomers thereof, decomposition products of polymer (P) and additives. Preferably, the total amount of bound vinylaromatic monomers in the composition (i.e. repeating units of the vinylaromatic monomers (M) in the polymer (P), in oligomers and co-oligomers of the vinylaromtic monomers (M)), constitutes at least 15 wt.%, more preferably at least 20 wt.%, more preferably at least 40 wt.%, more preferably at least 60 wt.%, based on the total weight of the composition. In some embodiments, the composition comprising polymer (P) is post-consumer waste comprising from 75 to 95% by weight, preferably from 80 to 90% by weight, based on a total weight of the post-consumer waste, of bound vinylaromatic monomers (M) in the form of repeating units of at least one polymer (P) and oligomers of the vinylaromatic monomers (M).

The amount of vinylaromatic monomers (M) in the pyrolysis oil (PO) obtained from the process of the invention will generally depend on the amount of repeating units of said vinylaromatic monomers (M) in the polymer (P) and on the amount of free vinylaromatic monomers (M) in the composition comprising the polymer (P) that is subjected to the pyrolysis of step a.. Typically, the amount of vinylaromatic monomers (M) in the pyrolysis oil (PO) is in the range of 60 to 80% by weight, often 65 to 75% by weight, based on the amount of bound vinylaromatic monomers (M) in the composition subjected to the pyrolysis of step a..

Furthermore, the composition may comprise monomers other than vinylaromatic monomers (M), oligomers of such monomers and/or co-oligomers of such monomers, preferably co-oligomers of such monomers with vinylaromatic monomers (M). Such other monomers are often selected from the group consisting of ethylene, alpha-olefins such as 1-propylene or 1-butylene, alkyl acrylates such as butyl acrylate, alkyl methacrylates, such as methyl methacrylate, dienes such as butadiene or isoprene, acrylonitrile, methacrylonitrile and vinyl chloride. Preferably, such other monomers, if present, are selected from the group consisting of butyl methacrylate, methyl methacrylate, acrylonitrile and butadiene. More preferably, such other monomers are selected from the group consisting of butyl methacrylate, acrylonitrile and butadiene.

Other components that may be present in the composition comprising polymer (P) are auxiliaries, additives and/or impurities, e.g. water, halogenated substances, inorganic or organic dyes or pigments, lubricants, waxes, amides of long chain organic acids, emulsifiers, soaps, paper, cardboard, metals, metal oxides, metal salts, fillers, and other additives such as UV stabilizers, hindered amine light stabilizers (HALS), hindered phenols, disulfite stabilizers, quenchers and absorbers.

The polymer (P) may be a polymer containing only repeating units derived from vinylaromatic monomers (M), preferably styrene, or a polymer containing repeating units derived from vinylaromatic monomers (M), preferably styrene, and repeating units derived from other monomers, such as the monomers listed above. Preferably, the polymer (P) comprises one or more polymers selected from the group consisting of polystyrene (PS), such as general purpose polystyrene (GPPS) or high impact polystyrene (HIPS), styrene acrylonitrile copolymers (SAN), acrylonitrile-styrene-acrylate copolymers (ASA), acrylonitrile-butadiene-styrene copolymers (ABS), styrene-butadiene-copolymers (SBC), styrene-isoprene copolymers (SIC), styrene-butadiene rubbers (SBR) and styrene-methyl methacrylate copolymers (SMMA). More preferably, the polymer (P) comprises one or more polymers selected from the group consisting of PS, HIPS, ASA, ABS and SBC.

Preferably, the polymer (P) comprises 30 to 100% by weight, more preferably 50 to 100% by weight, more preferably 60 to 100% by weight, more preferably 70 to 100% by weight, based on the total weight of polymer (P), of repeating units derived from vinyl aromatic monomers (M), preferably styrene or alpha-methylstyrene, more preferably styrene, and optionally 0 to 70% by weight, preferably 0 to 50% by weight, more preferably 0 to 40% by weight, more preferably 0 to 30% by weight, based on the total weight of polymer (P), of co-monomers, preferably selected from the group consisting of ethylene, alpha-olefins such as 1-propylene or 1-butylene, alkyl acrylates such as butyl acrylate, alkyl methacrylates, such as methyl methacrylate, dienes such as butadiene or isoprene, acrylonitrile, methacrylonitrile and vinyl chloride, more preferably selected from the group consisting of butyl methacrylate, methyl methacrylate, acrylonitrile and butadiene, more preferably selected from the group consisting of butyl methacrylate, acrylonitrile and butadiene.

Furthermore, when provided as part of a composition or polymer waste comprising polymer (P), the polymer (P) is preferably present in the composition or polymer waste in an amount of 30 to 100% by weight, more preferably 50 to 99.9% by weight, more preferably 60 to 99% by weight, more preferably 80 to 95% by weight, based on the total weight of the composition or polymer waste.

The pyrolysis of the polymer (P) may be carried out by any means known in the art. For example, the pyrolysis may be carried out in a fluidized bed reactor, tube reactor, tube bundle reactor, stirred tank reactor, extruder, or rotary kiln reactor. Preferably, the pyrolysis is carried out continuously, in a reactor allowing continuous pyrolysis, e.g. a fluidized bed reactor, tube reactor, tube bundle reactor, continuous stirred tank reactor, extruder or rotary kiln reactor.

Furthermore, the pyrolysis may be carried out under any conditions suitable for pyrolysis. Preferably, the pyrolysis is carried out at a temperature of from 350 to 1000 °C, preferably 400 to 900 °C, more preferably 500 to 800 °C, more preferably 550 to 650 °C, more preferably 580 to 630 °C. Furthermore, preferably the pyrolysis is carried out with an average residence time of the styrene-containing polymer waste stream in the reactor of from 0.1 second to 10 minutes, preferably from 0.2 seconds to 60 seconds, more preferably from 0.3 seconds to 10 seconds.

Other suitable conditions for pyrolysis are described, e.g. in WO2021/053074, WO2021/053075 and WO2021/074112.

A vapour (V) containing vinylaromatic monomers (M) and other components is obtained from the pyrolysis of the polymer (P) in step a. Where the vinylaromatic monomers (M) are styrene, the vapour (V) can be referred to as crude styrene gas (CSG).

For example, step a. may comprise following stages:
a1. providing a composition, preferably polymer waste, more preferably post-consumer polymer waste, comprising
   30 to 100% by weight, preferably 50 to 99.9% by weight, more preferably 60 to 99% by weight, more preferably 80 to 95% by weight, based on the total weight of the composition, of at least one polymer (P) containing repeating units of vinylaromatic monomers (M);
   0 to 60% by weight, preferably 0 to 45% by weight, more preferably 0 to 37% by weight, more preferably 0 to 18% by weight, based on the total weight of the composition, of polyolefins and/or polyalkyl(meth)acrylates;
   0 to 10% by weight, preferably 0 to 5% by weight, more preferably 0 to 3% by weight, more preferably 0 to 2% by weight, of other polymeric materials; and 0 to 40% by weight, preferably 0.1 to 35% by weight, more preferably 1 to 30% by weight, more preferably 5 to 20% by weight, based on the total weight of the composition, of additives and non-polymeric components;
a2. continuously feeding the composition provided in step a1. to the reaction zone of a pyrolysis reactor wherein the pyrolysis temperature is set to a value of from 350 to 1000 °C, preferably 400 to 900 °C, more preferably 500 to 800 °C, more preferably 550 to 650 °C, more preferably 580 to 630 °C and wherein the residence time of the composition in the reaction zone is set to a value of from 0.1 second to 10 minutes, preferably from 0.2 seconds to 60 seconds, more preferably from 0.3 seconds to 10 seconds, in order to conduct pyrolysis of the polymer (P) and other pyrolyzable components of the composition, and to obtain a vapour (V) containing vinylaromatic monomers (M);
a3. continuously withdrawing the vapour (V) containing vinylyromatic monomers (M) from the reaction zone of the pyrolysis reactor.

### Step b.

Step b. comprises the condensation of at least a portion of the monomers (M) from the vapour (V) by contacting the vapour (V) with an aqueous solution (A) or water. This leads to the termination of the radical pyrolysis reaction, and is also known as "quenching" of the reaction with the aqueous solution. Quenching induces the condensation of vinylaromatic monomers (M) and other condensable components present in the vapour (V), and transfers them to a liquid state. I.e. step b. includes both quenching and condensing.

The term "aqueous solution" in the context of the present invention relates to a homogeneous solution of any substance in water.

In one embodiment, using an aqueous solution (A) containing a phosphate salt for quenching the pyrolysis is preferable, since an additional step of adding a phosphate salt to the aqueous phase in step c. may be omitted. In another embodiment, it is preferable to add the phosphate afterwards in step c., between steps b. and c. or to apply a combination thereof. In yet another embodiment, the phosphate salt is added to the aqueous solution (A) used for quenching, and also added in step c. and/or between steps b. and c..

Where the quenching is carried out with an aqueous solution (A) of a phosphate salt, the aqueous solution (A) contains at least one phosphate salt in an amount leading to a phosphate (PO₄³⁻) concentration of > 0 mol/L, preferably at least 0.01 mol/L, more preferably at least 0.1 mol/L, more preferably 0.2 to 2 mol/L, more preferably 0.4 to 1 mol/L.

Quenching may be carried out by any suitable means known in the art, which involve direct contact of the vapour (V) comprising the pyrolysis products with an aqueous solution (A) or water. For example, quenching may be carried out by transferring the vapour (V) into a condensation vessel and introducing, preferably spraying, the aqueous solution (A) or water into the condensation vessel. Quenching may also be carried out by introducing the pyrolysis vapour (V) into the aqueous solution (A) or water ("bubbling") contained in a quenching vessel. Where quenching is carried out by introducing the pyrolysis vapour (V) into the aqueous solution (A) or water, it is often useful to lead the vapour (V) through a fine mesh, e.g. a glass frit, in order to decrease the size of bubbles, and thus increase the area of contact between the vapour (V) and the aqueous solution (A) or water.

Preferably, the conditions of quenching are selected thus that at least 60% by weight, more preferably at least 80% by weight, more preferably at least 90% by weight, more preferably at least 99% by weight of the vinylaromatic monomers (M) present in vapour (V) are condensed. This may be achieved, e.g., by selecting an appropriate temperature of the aqueous solution (A).

Preferably, the temperature of the aqueous solution (A) or water is set to a value of from -20 to 50 °C, more preferably from 5 to 40 °C, more preferably from 10 to 35 °C.

A heterogeneous liquid mixture of an organic phase, comprising vinylaromatic monomers (M), and an aqueous phase is obtained from the quenching of the pyrolysis reaction in step b.

### Step c.

Step c. comprises performing a phase separation between the organic phase comprising vinylaromatic monomers (M) and the aqueous phase.

To perform the inventive method, the phase separation between the organic phase and the aqueous phase must be carried out thus that the aqueous phase contains at least one phosphate salt in an amount leading to a phosphate (PO₄³⁻) concentration of at least 0.01 mol/L, more preferably at least 0.1 mol/L, more preferably 0.2 to 2 mol/L, more preferably 0.4 to 1 mol/L.

Accordingly, where an aqueous solution (A) not containing any phosphate salts or at a too low concentration, or demineralized water was used for quenching in step b., at least one phosphate salt is added to the heterogeneous mixture obtained from step b. to adjust the phosphate (PO₄³⁻) concentration to at least 0.01 mol/L, more preferably at least 0.1 mol/L, more preferably 0.2 to 2 mol/L, more preferably 0.4 to 1 mol/L, before phase separation in step c. is carried out. The phosphate salt may be provided as a solid, or may be dissolved prior to addition.

In contrast, in some embodiments where an aqueous solution (A) comprising a phosphate salt was used for quenching in step b., the addition of a phosphate salt in the phase separation step c. can be omitted. However, the phosphate may also be added between steps b. and c., or by applying a combination of an addition at said steps.

The phase separation between the organic and the aqueous phase may be carried out under any suitable conditions, at which the organic and aqueous phases are in the liquid state. Preferably, the phase separation is carried out at ambient pressure at 5 to 60 °C, more preferably at 10 to 50 °C, more preferably at 15 to 40 °C.

Furthermore, the phase separation may be carried out by any means known in the art for this purpose. For example, phase separation may be carried out directly in the vessel used for quenching.

Alternatively, the heterogeneous mixture may be transferred into a separate vessel, e.g. a separating funnel or a phase separator, where the phase separation is carried out. In continuous processes, the heterogeneous mixture obtained from step b. may e.g. be continuously transferred into a vessel (if required together with a stream of one or more phosphate salts) where the organic phase may be continuously withdrawn from the top of the vessel and the aqueous phase may be continuously withdrawn from the bottom of the vessel, while the mixed phase is continuously provided in the center of the vessel. In cases where the organic phase contains a large amount of high-density organic components (e.g. halogenated organic components, such as halogenated vinylaromatic monomers), the withdrawal of the aqueous phase may also be carried out at the top of the vessel, while the withdrawal of the organic phase is carried out at the bottom of the vessel. Where the organic phase is rich in styrene or low-density components, the organic phase is typically withdrawn from the top of the vessel.

When performing the phase separation of step c., the volume ratio of organic phase to the aqueous phase may for example be from 95:5 to 5:95, preferably from 80:20 to 10:90, more preferably from 50:50 to 20:80, more preferably from 40:60 to 25:75. The ratio may e.g. be adjusted by regulating the amount of aqueous solution (A) or water used in step b. or by adding and withdrawing aqueous solution in step c..

In order to perform the method of the invention, the aqueous phase comprises a phosphate salt. The amount of phosphate salt is selected such that the concentration of phosphate ions PO₄³⁻, is at least 0.01 mol/L, more preferably at least 0.1 mol/L, more preferably 0.2 to 2 mol/L, more preferably 0.4 to 1 mol/L.

The phosphate salt used in the inventive method may be any phosphate salt with sufficient solubility in water to obtain the above concentrations. Preferably the salt is selected from alkali metal phosphates, alkali metal phosphate hydrates, ammonium phosphates, ammonium phosphate hydrates, and mixtures thereof, preferably selected from the group consisting of NaₙH₃₋ₙPO₄, KₙH₃₋ₙPO₄, (NH₄)ₙH₃₋ₙPO₄, and their hydrates, wherein n is an integer from 1 to 3, and mixtures thereof, more preferably selected from Na₃PO₄, K₃PO₄, (NH₄)₃PO₄, and their hydrates, and mixtures thereof, more preferably selected from Na₃PO₄, K₃PO₄ and mixtures thereof, more preferably K₃PO₄.

### Step d.

In step d., the organic phase containing vinylaromatic monomers (M) is collected from the phase-separated heterogeneous mixture, to obtain the pyrolysis oil (PO) containing vinylaromatic monomers (M).

The collecting may be carried out by any suitable means known in the art. For example, where a separating funnel was used for phase separation, if the organic phase has a lower density than the aqueous phase, the aqueous phase can be released from the bottom of the separating funnel, while the organic phase comprising vinylaromatic monomers (M) is collected in the separating funnel. If the organic phase has a higher density than the aqueous phase (e.g. when halogenated organic compounds such as halogenated vinylaromatic monomers are present), the organic phase may be released from the bottom of the separating funnel and collected in a separate vessel, whereas the aqueous phase may remain in the separating funnel.

Alternatively, if the organic phase has a lower density than the aqueous phase, the organic phase comprising vinylaromatic monomers (M) may be siphoned from the top of the phase-separated heterogeneous mixture and be collected in a separate vessel, whereas the aqueous phase may remains in the vessel used for phase separation. If the organic phase has a higher density than the aqueous phase (e.g. when halogenated organic compounds such as halogenated vinylaromatic monomers are present), the organic phase may be collected in the vessel used for phase separation, whereas the aqueous phase us siphoned from the top.

In continuous processes, if the organic phase has a lower density than the aqueous phase, the organic phase may e.g. be continuously withdrawn from the top of the vessel, wherein phase separation of step c. took place, and further be collected in a separate vessel, e.g. in a storage tank, whereas the aqueous phase may be continuously withdrawn from the bottom of the vessel. If the organic phase has a higher density than the aqueous phase (e.g. when halogenated organic compounds such as halogenated vinylaromatic monomers are present), the organic phase may e.g. be continuously withdrawn from the bottom of the vessel, wherein phase separation of step c. took place, and further be collected in a separate vessel, e.g. in a storage tank, whereas the aqueous phase may be continuously withdrawn from the top of the vessel.

Preferably, simultaneously with the collection of the organic phase, the aqueous phase is also collected and further recycled for use in the quenching step b.

In addition to the above steps a. to d., the pyrolysis oil (PO) containing vinylaromatic monomers (M) obtained from step d. may further be processed, in order to purify and/or isolate the pyrolysis oil (PO) or its individual components, such as the vinylaromatic monomers (M), preferably styrene and/or alpha-methylstyrene, in particular styrene, and optionally side products, e.g. ethylbenzene or toluene. Preferably, the pyrolysis oil (PO) is further processed by distillation, more preferably fractional distillation.

In continuous processes, the pyrolysis oil (PO) may e.g. be fed into a fractional distillation column, where it is fractionally distilled to obtain fractions enriched with individual components of the pyrolysis oil (PO), preferably at least one fraction enriched with vinylaromatic monomers (M), in particular styrene. In some embodiments, a multitude of distillation steps can be performed in order to obtain components of high purity, e.g. at least 99 % by weight. In addition or alternatively, purification of the pyrolysis oil (PO) can involve crystallization, e.g. fractional crystallization, by means known in the art.

The pyrolysis oil (PO) obtained from the process of the invention typically contains at least 10 wt.-%, often at least 40 wt.%, often at least 50 wt.%, in some cases at least 60% by weight, based on the total weight of the pyrolysis oil (PO), of vinylaromatic monomers (M), in particular styrene.

In a preferred embodiment, the steps of phase separation (step c.), collection (step d.) and optional distillation are carried out until the produced pyrolysis oil (PO), obtained by performing the method of the invention, contains at least 70 wt.% of vinylaromatic monomers (M), in particular styrene. In another embodiment the steps are carried out until the mixture of vinylaromatic monomers (M), in the produced pyrolysis oil (PO), obtained by performing the inventive method, contains at least 50 wt.%, preferably at least 70 wt.%, based on the mixture of vinylaromatic monomers (M), of styrene.

Another aspect of the disclosed invention is a system configured for performing the inventive method for the recovery or recycling of vinylaromatic monomers (M), in particular styrene, from a polymer (P) as described above, or a composition comprising polymer (P). The device comprises a means for carrying out a pyrolysis of a polymer (P) comprising repeating units of vinylaromatic monomers (M), a means for quenching the pyrolysis reaction with an aqueous solution (A) or water, a means for performing phase separation, a means for collecting the organic phase from the means for performing phase separation, and a means for dosing salt, configured to dose at least one phosphate salt into the aqueous solution (A) used for quenching the pyrolysis reaction or into the means for performing phase separation. Preferably, the system further comprises a distillation means. The means for carrying out the individual steps are preferably those described above for the individual steps.

The invention is further illustrated by the following examples, Figures and claims.
Fig. 1 shows a graph of phase separation over time in heterogeneous mixtures of pyrolysis oil and aqueous solutions of K₃PO₄ with different concentrations from 0 to 1 M, as measured in Example B.
Fig. 2 shows a graph of phase separation after 5 min for heterogeneous mixtures of pyrolysis oil and aqueous solutions of K₃PO₄ with different concentrations from 0 to 1 M, as measured in Example B.
Fig. 3 shows a graph of the reproducibility of phase separation for concentrations of 0.1 M and 0.4 M, as measured in Example B.
Fig. 4 shows a graph comparing phase separation using Na₃PO₄ and K₃PO₄, as examined in Example C.
Fig. 5 shows the setup for a pyrolysis process as described in Example D.

### Examples

The examples were carried out using pyrolysis oil (PO) obtained from a standard pyrolysis process of post-consumer waste containing 85 wt% of bound styrene monomers in the polymer chains. The aqueous solutions were based on demineralized water, wherein different amounts of salts were dissolved.

An experimental set-up was installed to allow for the monitoring of the described separation of layers during the phase separation process via camera. A measuring ruler was placed vertically next to the glass vial to allow for a quantification of the phase separation degree in a function of time.

A glass vial was successively filled with demineralized water or an aqueous solution containing K₃PO₄, Na₃PO₄ or KCl in a specific quantity, and the pyrolysis oil (PO). Aqueous solutions were obtained by dissolving the corresponding solid salts in demineralized water. (K₃PO₄·3H₂O and Na₃PO₄·12H₂O were used to prepare K₃PO₄ and Na₃PO₄ solutions, respectively. The water presence in these solids was taken into consideration when determining the concentration of the obtained solutions.)

For the experiment, the oil was added slowly into the glass vial via syringe against the inner wall of the vial, so as not to upset the obtained two-layer-system. The glass vial was then sealed with an aluminium crimp cap and placed into the experimental setup.

A first photograph was taken to determine the original height of the contact line between both layers. The vial was then vigorously shaken by hand for 5 s, resulting in a heterogeneous emulsion, and was immediately placed in the setup after which a video recording was started. After 5 min, the recording was stopped, and a second photograph was taken with additional lighting to show a possible emulsion more clearly.

The obtained phase separation was quantified as follows: During phase separation, a homogeneous aqueous fraction appears, starting at the bottom of the glass vial.

As the emulsion is gradually broken up, this layer rises from the bottom (0 %) to the height of the original contact line before mixing (100 %). At complete (100 %) phase separation, the original height is reached, while a partial separation (between 0 % and 100 %) reaches a height between the bottom of the glass vial and the original height.

When performing the experiments, it has to be taken into account that phase separation depends on many factors. E.g., phase separation time depends on the geometry of the container, where a bigger interface surface usually phase separates faster than a smaller interface surface. In addition, the amount of aqueous phase and the amount of organic phase, i.e. the ratio of the two phases, influences the progress of phase separation. More water allows more emulsion formation and thus, generates longer distances of the emulsion to phase separate, whereas a higher amount of organic phase allows deeper infusion into the aqueous phase during mixing. Therefore, the same vials were used for all experiments, and the same volume ratios of pyrolysis oil (PO) and aqueous solution were employed. The effects shown in the examples are observed for other configurations, provided the vials and ratios in the comparative examples are the same as in the examples according to the invention. Hence relative comparison is allowed.

### Example A

In a first approach, one type of glass vial was chosen and the influence of the ratio between aqueous phase and pyrolysis oil (PO) on phase separation within a 5 min timeframe was observed. 15 mL aqueous solution (demineralized water or 1 M K₃PO₄ solution; 1 M = 1 mol/L) and 5 to 15 mL PO were filled into a glass vial of 50 mL (internal diameter = 28.6 mm), mixed for 5 s and then allowed to phase separate for 5 min at room temperature (20 °C).

**Table 1:**

| Phase separation between PO and aqueous phase in different ratios after 5 min | | | | |
|---|---|---|---|---|
| | volume [mL] | | composition | phase separation after 5 min [%] |
| | PO | aqueous solution | | |
| 1 | 15 | 15 | demineralized water | 0 |
| 2 | 15 | 15 | 1 mol/L K₃PO₄ | 8.0 |
| 3 | 7.5 | 15 | demineralized water | 0 |
| 4 | 7.5 | 15 | 1 mol/L K₃PO₄ | 98.0 |
| 5 | 5 | 15 | demineralized water | 0 |
| 6 | 5 | 15 | 1 mol/L K₃PO₄ | 100.0 |

While in mixtures with demineralized water no phase separation occurred during the observation time of 5 min (and even not after 24 hours, not shown in tables), immediate phase separation up to 100 % after 5 min was measured with K₃PO₄ aqueous solution. Upon these results, a volume of 5 mL PO and 15 mL aqueous solution, i. e. a ratio of 1:3, was selected for further experiments to allow evaluation of the enhanced phase separation effect within the observation time of 5 min.

### Example B

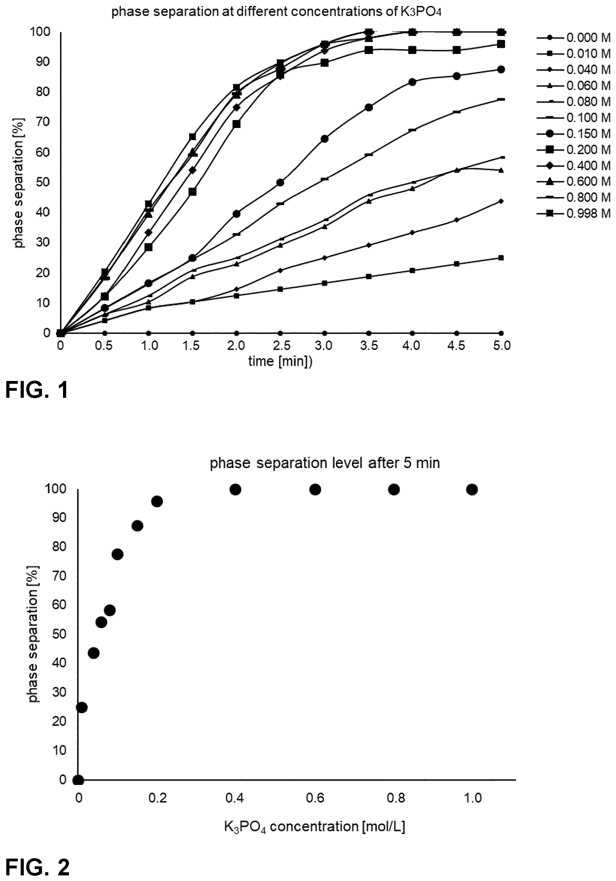

The mixture of demineralized water and PO after shaking does not separate into two distinct layers over a period of 5 min but forms one overall phase, a heterogeneous emulsion. It was found that the presence of phosphate ions enhances the rate of phase separation. Hence, in a second approach, the effect of increasing the PO₄³⁻ concentration in the aqueous layer on phase separation was analysed. The experiment was conducted as outlined above: 5 mL PO and 15 mL aqueous solution with various concentrations of K₃PO₄ were mixed for 5 s and then, phase separation at room temperature was observed for 5 min. Measurements were taken at several time points after mixing (table 2, after 2.5 and 5 min; Fig. 1, every 30 s from 0.5 to 5 min). The results are shown in Table 2 and Fig. 1. Fig. 2 presents the dependency of phase separation [%] after 5 min on the concentration of the K₃PO₄ aqueous solution; concentrations vary from 0 to 1 mol/L of K₃PO₄.

**Table 2: phase separation between CSO and aqueous phase with different concentrations of K₃PO₄**

| | type of example | aqueous solution | concentration [mol/L] | phase separation [%] | |
|---|---|---|---|---|---|
| | | | | after 2.5 min | after 5 min |
| 5 | comparative | demineralized water | 0 | 0 | 0 |
| 7 | inventive | K₃PO₄ | 0.01 | 14.6 | 25.0 |
| 8 | inventive | K₃PO₄ | 0.04 | 20.8 | 43.8 |
| 9 | inventive | K₃PO₄ | 0.06 | 29.2 | 54.2 |
| 10 | inventive | K₃PO₄ | 0.08 | 31.3 | 58.3 |
| 11 | inventive | K₃PO₄ | 0.10 | 42.9 | 77.6 |
| 12 | inventive | K₃PO₄ | 0.15 | 50.0 | 87.5 |
| 13 | inventive | K₃PO₄ | 0.20 | 85.7 | 95.5 |
| 14 | inventive | K₃PO₄ | 0.40 | 85.4 | 100.0 |
| 15 | inventive | K₃PO₄ | 0.60 | 89.6 | 100.0 |
| 16 | inventive | K₃PO₄ | 0.80 | 87.8 | 100.0 |
| 6 | inventive | K₃PO₄ | 1.00 | 89.8 | 100.0 |

The results clearly demonstrate the beneficial effect of K₃PO₄ in the aqueous layer to increase the rate of phase separation between PO and the aqueous solution. A saturation of the effect is reached as from a concentration of approximately 0.4 mol/L K₃PO₄ in the aqueous solution.

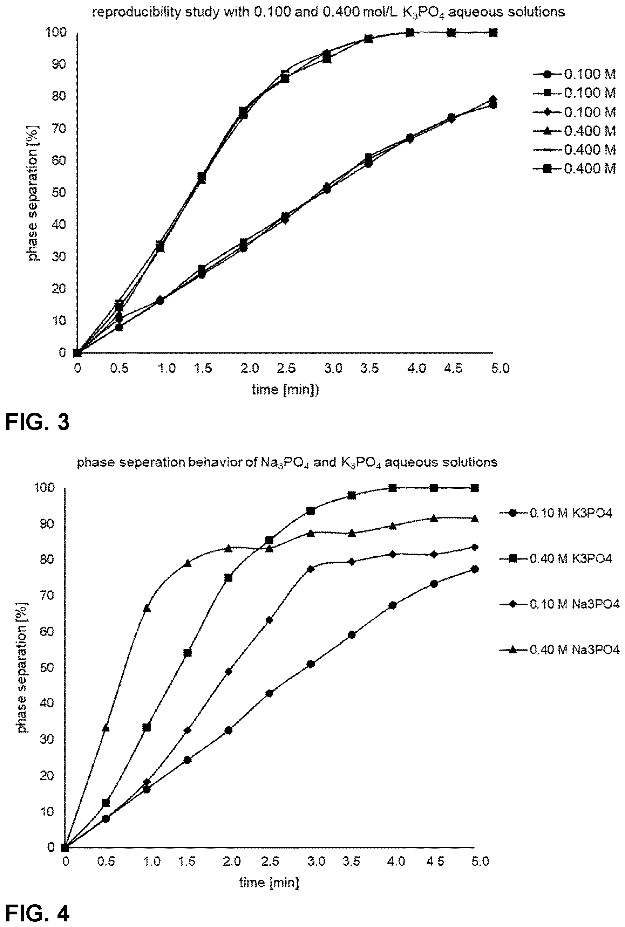

To confirm the findings of the experiment, measurements were repeated several times. All results showed good reproducibility as shown in Fig. 3 and listed in Table 3 for concentrations of 0.1 and 0.4 mol/L K₃PO₄.

**Table 3: reproducibility of phase separation between CSO and aqueous phase with 0.1 or 0.4 mol/L K₃PO₄**

| | type of example | aqueous solution | concentration [mol/L] | phase separation [%] | |
|---|---|---|---|---|---|
| | | | | after 2.5 min | after 5 min |
| 11 | inventive | K₃PO₄ | 0.10 | 42.9 | 77.6 |
| 17 | inventive | K₃PO₄ | 0.10 | 42.9 | 77.6 |
| 18 | inventive | K₃PO₄ | 0.10 | 41.7 | 79.2 |
| 14 | inventive | K₃PO₄ | 0.40 | 85.4 | 100.0 |
| 19 | inventive | K₃PO₄ | 0.40 | 87.8 | 100.0 |
| 20 | inventive | K₃PO₄ | 0.40 | 85.7 | 100.0 |

### Example C

In further approaches, others salt solutions were tested in the same setup, in order to confirm that the active ingredient of the fast-separating mixture is indeed the phosphate (PO₄-³⁻). The faster phase separation could either be caused by the anionic PO₄³⁻ (phosphate) ions or the cationic K⁺ ions of the K₃PO₄. Therefore, the experimental result was compared both to Na₃PO₄ and to KCI as ingredients in the aqueous solution. The previously used 0.10 M and 0.40 M K₃PO₄ solutions were chosen as reference.

In case of the use of KCI aqueous solutions, concentrations of 0.30 M and 1.2 M were employed, in order to establish the same K⁺ ion concentration as in the 0.10 M and 0.4 M K₃PO₄ solutions, respectively.

Measurements according to the experimental setup were not possible, as during the observation a clear horizontal interface between the layers was not formed. Even though some separation occurred, it could rather be described as an emulsion of larger PO droplets in the aqueous solution. This leads to the conclusion that the effect described above is not caused by the K⁺ ions.

In case of the Na₃PO₄ aqueous solution, phase separation was observed in a similar way as for K₃PO₄ before. In both cases, a clear horizontal interface was achieved during phase separation. This leads to the conclusion that the presence of PO₄³⁻ ions is responsible for the improved and accelerated phase separation, and the cation can be varied. The results are shown below in table 4 and in Fig. 4.

**Table 4: phase separation between CSO and aqueous phase with K₃PO₄ or Na₃PO₄ aqueous solution**

| | type of example | aqueous solution | concentration [mol/L] | phase separation [%] | |
|---|---|---|---|---|---|
| | | | | after 2.5 min | after 5 min |
| 11 | inventive | K₃PO₄ | 0.10 | 42.9 | 77.6 |
| 21 | inventive | Na₃PO₄ | 0.10 | 63.3 | 83.7 |
| 14 | inventive | K₃PO₄ | 0.40 | 85.4 | 100.0 |
| 22 | inventive | Na₃PO₄ | 0.40 | 83.3 | 91.7 |

The above findings also show that, although the mass of the used salts, and thus the density of the aqueous solution, also may have some influence on phase separation (Na₃PO₄: 0.4 mol/L = 65.6 g/L; K₃PO₄: 0.4 mol/L = 84.9 g/L; KCl: 1.2 mol/L = 89.5 g/L), the main effect can be attributed to the presence of PO₄³⁻ ions.

### Example D

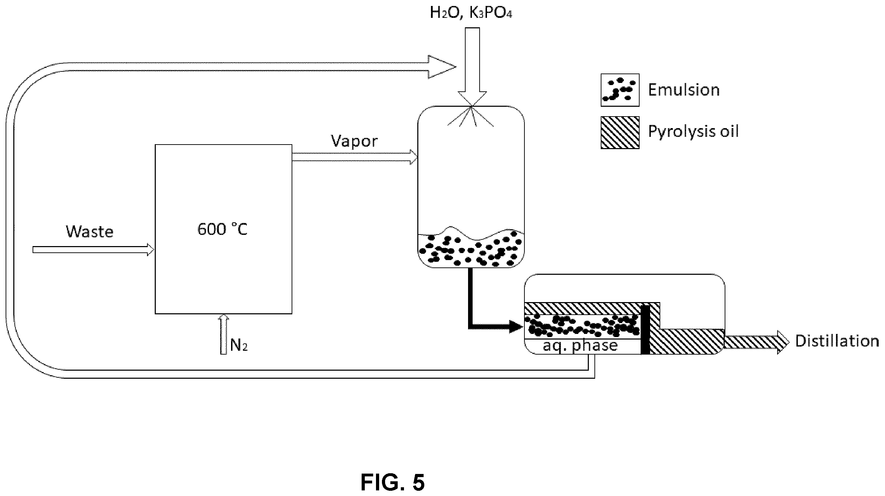

Pyrolysis of post-consumer waste containing 85 wt% of bound styrene monomers in the polymer chains is carried out in a standard fluidizing bed reactor configured for carrying out continuous pyrolysis of polystyrene, operating at an inner temperature of 600 °C. The feeding rate of polystyrene into the pyrolysis zone of the reactor is selected to ensure an average residence time of polystyrene in the pyrolysis zone of 5 seconds. The pyrolysis vapours are transferred via a conduit into a quencher, where a 0.4 M aqueous K₃PO₄ solution having a temperature of 20 °C is sprayed from the top into the condensation vessel. The condensed phase is collected at the bottom of the vessel and transferred into a phase separator. The organic phase is withdrawn from the top of the phase separator and subjected to distillation in a fractional distillation column. The aqueous phase is withdrawn from the bottom of the vessel and recycled into the condensation vessel. The setup is schematically shown in Fig. 5.

## Claims

1. A method for producing a pyrolysis oil (PO) containing vinylaromatic monomers (M) from a polymer (P) comprising repeating units of said monomers (M), wherein the method comprises the steps:
a. Pyrolysis of the polymer (P) comprising repeating units of vinylaromatic monomers (M), to obtain a vapour (V) containing said monomers (M);
b. Condensing at least a portion of the monomers (M) by contacting the vapour (V) with an aqueous solution (A) or water, to obtain a heterogeneous mixture of an organic phase containing vinylaromatic monomers (M) and an aqueous phase;
c. Performing a phase separation between the organic phase and the aqueous phase; and
d. Collecting the organic phase containing vinylaromatic monomers (M), to obtain the pyrolysis oil (PO) containing vinylaromatic monomers (M),
wherein in step c. the aqueous phase contains at least one phosphate salt in an amount leading to a phosphate (PO₄³⁻) concentration of at least 0.01 mol/L, more preferably at least 0.1 mol/L, more preferably 0.2 to 2 mol/L, more preferably 0.4 to 1 mol/L.

2. The method according to claim 1, wherein the method is continuous.

3. The method according to claim 1 or 2, wherein the vinylaromatic monomers (M) comprise, preferably consist of, styrene.

4. The method according to any one of claims 1 to 3, wherein the polymer (P) originates from polymer waste, preferably post-consumer polymer waste.

5. The method according to any one of claims 1 to 4, wherein the method further comprises an additional step, preferably a distillation step, wherein the pyrolysis oil (PO) or individual components thereof are purified and/or isolated.

6. The method according to any one of claims 1 to 5, wherein in step c., the volume ratio of the organic phase to the aqueous phase is from 95:5 to 5:95, preferably from 80:20 to 10:90, more preferably from 50:50 to 20:80, more preferably from 40:60 to 25:75.

7. The method according to any one of claims 1 to 6, wherein the at least one phosphate salt is introduced into the aqueous solution (A) used in step b. for condensing monomers (M), or is introduced into the heterogeneous mixture between step b. and c., or in step c. after obtaining the heterogeneous mixture in step b., or a combination of these options.

8. The method according to any one of claims 1 to 7, wherein the phosphate salt is selected from alkali metal phosphates, ammonium phosphates, their hydrates and mixtures thereof, preferably selected from the group consisting of NaₙH₃₋ₙPO₄, KₙH₃₋ₙPO₄, (NH₄)ₙH₃₋ₙPO₄, and their hydrates, wherein n is an integer from 1 to 3, and mixtures thereof, more preferably selected from Na₃PO₄, K₃PO₄, (NH₄)₃PO₄, and their hydrates, and mixtures thereof, more preferably selected from Na₃PO₄, K₃PO₄, their hydrates and mixtures thereof, more preferably K₃PO₄ and its hydrates.

9. The method according to any one of claims 1 to 8, wherein the phase separation is carried out at 5 to 60 °C, preferably at 10 to 50 °C, more preferably at 15 to 40 °C.

10. The method according to any of claims 1 to 9, wherein step a. comprises the following stages:
a1. providing a composition, preferably polymer waste, comprising 30 to 100% by weight, preferably 50 to 99.9% by weight, more preferably 60 to 99% by weight, more preferably 80 to 95% by weight, based on the total weight of the composition, of at least one polymer (P) containing repeating units of vinylaromatic monomers (M);
0 to 60% by weight, preferably 0 to 45% by weight, more preferably 0 to 37% by weight, more preferably 0 to 18% by weight, based on the total weight of the composition, of polyolefins and/or polyalkyl(meth)acrylates;
0 to 10% by weight, preferably 0 to 5% by weight, more preferably 0 to 3% by weight, more preferably 0 to 2% by weight, of other polymeric materials; and
0 to 40% by weight, preferably 0.1 to 35% by weight, more preferably 1 to 30% by weight, more preferably 5 to 20% by weight, based on the total weight of the composition, of additives and non-polymeric components;
a2. continuously feeding the composition provided in step a1. to the reaction zone of a pyrolysis reactor wherein the pyrolysis temperature is set to a value of from 350 to 1000 °C, preferably 400 to 900 °C, more preferably 500 to 800 °C, more preferably 550 to 650 °C, more preferably 580 to 630 °C and wherein the residence time of the composition in the reaction zone is set to a value of from 0.1 second to 10 minutes, preferably from 0.2 seconds to 60 seconds, more preferably from 0.3 seconds to 10 seconds, in order to conduct pyrolysis of the polymer (P) and other pyrolyzable components of the composition, and to obtain a vapour (V) containing vinylaromatic monomers (M);
a3. continuously withdrawing the vapour (V) containing vinylyromatic monomers (M) from the reaction zone of the pyrolysis reactor.

11. A system configured for performing the method of any one of claims 1 to 10, comprising a means for carrying out a pyrolysis of a polymer (P) comprising repeating units of vinylaromatic monomers (M), a means for quenching the pyrolysis reaction with an aqueous solution (A), a means for performing phase separation, a means for collecting the organic phase from the means for performing phase separation, and a means for dosing salt, configured to dose at least one phosphate salt into the means for performing phase separation, and optionally a distillation means.

12. The use of an aqueous solution (A) containing a phosphate salt in an amount leading to a phosphate (PO₄³⁻) concentration of > 0 mol/L preferably at least 0.01 mol/L, more preferably at least 0.1 mol/L, more preferably 0.2 to 2 mol/L, more preferably 0.4 to 1 mol/L for quenching a pyrolysis reaction for the recovery of vinylaromatic monomers (M) from a polymer (P) comprising repeating units of said vinylaromatic monomers (M).

13. A method for increasing the speed of phase separation of a heterogeneous liquid mixture comprising vinylaromatic monomers (M) obtained from pyrolysis of a polymer (P), and an aqueous solution (A), wherein the process comprises the step of adjusting the amount of phosphate ions in the aqueous solution (A) to a concentration of at least 0.01 mol/L, preferably of at least 0.1 mol/L, more preferably of 0.2 to 2 mol/L, more preferably of 0.4 mol/L to 1 mol/L.

14. The use of an aqueous solution (A) containing a phosphate salt in an amount leading to a phosphate (PO₄³⁻) concentration of > 0 mol/L preferably at least 0.01 mol/L, more preferably at least 0.1 mol/L, more preferably 0.2 to 2 mol/L, more preferably 0.4 to 1 mol/L, for washing a pyrolysis oil (PO) containing vinylaromatic monomers (M) obtained from pyrolysis of a polymer (P) comprising repeating units of said monomers (M).

15. A heterogeneous liquid mixture comprising an organic phase comprising vinylaromatic monomers (M) obtained from pyrolysis of a polymer (P), and an aqueous phase comprising at least one phosphate salt in an amount leading to a phosphate (PO₄³⁻) concentration of at least 0.01 mol/L, preferably of at least 0.1 mol/L, more preferably 0.2 to 2 mol/L, more preferably 0.4 to 1 mol/L.

## Patentansprüche

1. Verfahren zur Herstellung eines Pyrolyseöls (PO) enthaltend vinylaromatische Monomere (M) aus einem Polymer (P) umfassend Wiederholungseinheiten dieser Monomere (M), wobei das Verfahren die Schritte umfasst:
a. Pyrolyse des Polymers (P) umfassend Wiederholungseinheiten der vinylaromatischen Monomere (M), um einen Dampf (V) enthaltend die Monomere (M) zu erhalten;
b. Kondensieren mindestens eines Teils der Monomere (M) durch Inkontaktbringen des Dampfes (V) mit einer wässrigen Lösung (A) oder Wasser, um ein heterogenes Gemisch aus einer organischen Phase enthaltend vinylaromatische Monomere (M) und einer wässrigen Phase zu erhalten;
c. Durchführen einer Phasentrennung zwischen der organischen Phase und der wässrigen Phase; und
d. Sammeln der organischen Phase enthaltend vinylaromatische Monomere (M), um das Pyrolyseöl (PO) enthaltend vinylaromatische Monomere (M) zu erhalten,
wobei in Schritt c) die wässrige Phase mindestens ein Phosphatsalz in einer Menge enthält, die zu einer Phosphatkonzentration (PO₄³⁻) von mindestens 0,01 mol/L, bevorzugt mindestens 0,1 mol/L, mehr bevorzugt 0,2 bis 2 mol/L, mehr bevorzugt 0,4 bis 1 mol/L führt.

2. Verfahren nach Anspruch 1, wobei das Verfahren kontinuierlich ist.

3. Verfahren nach Anspruch 1 oder 2, wobei die vinylaromatischen Monomere (M) Styrol umfassen, bevorzugt aus Styrol bestehen.

4. Verfahren nach einem der Ansprüche 1 bis 3, wobei das Polymer (P) aus Polymerabfall, bevorzugt aus post-Verbraucher-Polymerabfall, stammt.

5. Verfahren nach einem der Ansprüche 1 bis 4, wobei das Verfahren ferner einen zusätzlichen Schritt, bevorzugt einen Destillationsschritt, umfasst, in dem das Pyrolyseöl (PO) oder einzelne Bestandteile davon gereinigt und/oder isoliert werden.

6. Verfahren nach einem der Ansprüche 1 bis 5, wobei in Schritt c das Volumenverhältnis der organischen Phase zur wässrigen Phase von 95:5 bis 5:95, bevorzugt von 80:20 bis 10:90, mehr bevorzugt von 50:50 bis 20:80, mehr bevorzugt von 40:60 bis 25:75 ist.

7. Verfahren nach einem der Ansprüche 1 bis 6, wobei das mindestens eine Phosphatsalz in die in Schritt b. zur Kondensation von Monomeren (M) verwendete wässrige Lösung (A) eingebracht wird oder in das heterogene Gemisch zwischen Schritt b. und c. eingebracht wird oder in Schritt c. nach Erhalt des heterogenen Gemischs in Schritt b. eingebracht wird, oder eine Kombination dieser Optionen.

8. Verfahren nach einem der Ansprüche 1 bis 7, wobei das Phosphatsalz aus Alkalimetallphosphaten, Ammoniumphosphaten, deren Hydraten und Mischungen davon ausgewählt ist, bevorzugt ausgewählt ist aus der Gruppe bestehend aus NaₙH₃₋ₙPO₄, KₙH₃₋ₙPO₄, (NH₄)ₙH₃₋ₙPO₄ und deren Hydraten, wobei n eine ganze Zahl von 1 bis 3 ist, und Mischungen davon, mehr bevorzugt ausgewählt ist aus Na₃PO₄, K₃PO₄, (NH₄)₃PO₄ und deren Hydraten, und Mischungen davon, mehr bevorzugt ausgewählt ist aus Na₃PO₄, K₃PO₄, deren Hydraten, und Mischungen davon, mehr bevorzugt K₃PO₄ und dessen Hydraten.

9. Verfahren nach einem der Ansprüche 1 bis 8, wobei die Phasentrennung bei 5 bis 60 °C, bevorzugt bei 10 bis 50 °C, mehr bevorzugt bei 15 bis 40 °C durchgeführt wird.

10. Verfahren nach einem der Ansprüche 1 bis 9, wobei Schritt a. die folgenden Stufen umfasst:
a1. Bereitstellen einer Zusammensetzung, bevorzugt Polymerabfall, umfassend 30 bis 100 Gew.-%, bevorzugt 50 bis 99,9 Gew.-%, mehr bevorzugt 60 bis 99 Gew.-%, mehr bevorzugt 80 bis 95 Gew.-%, bezogen auf das Gesamtgewicht der Zusammensetzung, mindestens eines Polymers (P) umfassend Wiederholungseinheiten von vinylaromatischen Monomeren (M);
0 bis 60 Gew.-%, bevorzugt 0 bis 45 Gew.-%, mehr bevorzugt 0 bis 37 Gew.-%, mehr bevorzugt 0 bis 18 Gew.-%, bezogen auf das Gesamtgewicht der Zusammensetzung, an Polyolefinen und/oder Polyalkyl(meth)acrylaten;
0 bis 10 Gew.-%, bevorzugt 0 bis 5 Gew.-%, mehr bevorzugt 0 bis 3 Gew.-%, mehr bevorzugt 0 bis 2 Gew.-%, bezogen auf das Gesamtgewicht der Zusammensetzung, an anderen polymeren Materialien; und
0 bis 40 Gew.-%, bevorzugt 0,1 bis 35 Gew.-%, mehr bevorzugt 1 bis 30 Gew.-%, mehr bevorzugt 5 bis 20 Gew.-%, bezogen auf das Gesamtgewicht der Zusammensetzung, an Additiven und nicht-polymeren Komponenten;
a2. kontinuierliches Zuführen der in Schritt a1 bereitgestellten Zusammensetzung in die Reaktionszone eines Pyrolysereaktors, in dem die Pyrolysetemperatur auf einen Wert von 350 bis 1000 °C, bevorzugt 400 bis 900 °C, mehr bevorzugt 500 bis 800 °C, mehr bevorzugt 550 bis 650 °C, mehr bevorzugt 580 bis 630 °C eingestellt ist und wobei die Verweilzeit der Zusammensetzung in der Reaktionszone auf einen Wert von 0,1 Sekunden bis 10 Minuten, bevorzugt von 0,2 Sekunden bis 60 Sekunden, mehr bevorzugt von 0,3 Sekunden bis 10 Sekunden eingestellt ist, um eine Pyrolyse des Polymers (P) und anderer pyrolysierbarer Bestandteile der Zusammensetzung durchzuführen und einen Dampf (V) enthaltend vinylaromatische Monomere (M) zu erhalten;
a3. kontinuierliches Abziehen des vinylaromatische Monomere (M) enthaltenden Dampfes (V) aus der Reaktionszone des Pyrolysereaktors.

11. System, das zur Durchführung des Verfahrens nach einem der Ansprüche 1 bis 10 eingerichtet ist, umfassend ein Mittel zur Durchführung einer Pyrolyse eines Polymers (P) umfassend Wiederholungseinheiten aus vinylaromatischen Monomeren (M), ein Mittel zum Quentschen der Pyrolysereaktion mit einer wässrigen Lösung (A), ein Mittel zur Durchführung einer Phasentrennung, ein Mittel zum Sammeln der organischen Phase aus dem Mittel zur Durchführung der Phasentrennung und ein Mittel zur Dosierung von Salz, die eingerichtet ist, mindestens ein Phosphatsalz in das Mittel zur Durchführung der Phasentrennung zu dosieren, und optional ein Destillationsmittel.

12. Verwendung einer wässrigen Lösung (A) enthaltend ein Phosphatsalz in einer Menge, die zu einer Phosphatkonzentration (PO₄³⁻) von > 0 mol/L, bevorzugt mindestens 0,01 mol/L, mehr bevorzugt mindestens 0,1 mol/L, mehr bevorzugt 0,2 bis 2 mol/L, mehr bevorzugt 0,4 bis 1 mol/L führt, zum Quenstschen einer Pyrolysereaktion zur Wiedergewinnung von vinylaromatischen Monomeren (M) aus einem Polymer (P), umfassend Wiederholungseinheiten dieser vinylaromatischen Monomere (M).

13. Verfahren zum Erhöhen der Geschwindigkeit der Phasentrennung eines heterogenen Flüssigkeitsgemisches umfassend vinylaromatische Monomere (M), die durch Pyrolyse eines Polymers (P) erhalten wurden, und einer wässrigen Lösung (A), wobei das Verfahren den Schritt umfasst, die Menge an Phosphationen in der wässrigen Lösung (A) auf eine Konzentration von mindestens 0,01 mol/L, bevorzugt von mindestens 0,1 mol/L, mehr bevorzugt von 0,2 bis 2 mol/L, mehr bevorzugt von 0,4 mol/L bis 1 mol/L einzustellen.

14. Verwendung einer wässrigen Lösung (A) enthaltend ein Phosphatsalz in einer Menge, die zu einer Phosphatkonzentration (PO₄³⁻) von > 0 mol/L, bevorzugt mindestens 0,01 mol/L, mehr bevorzugt mindestens 0,1 mol/L, mehr bevorzugt 0,2 bis 2 mol/L, mehr bevorzugt 0,4 bis 1 mol/L führt, zum Waschen eines Pyrolyseöls (PO) enthaltend vinylaromatische Monomere (M), die aus der Pyrolyse eines Polymers (P) umfassend Wiederholungseinheiten dieser Monomere (M) erhalten wurden.

15. Heterogenes Flüssigkeitsgemisch, umfassend eine organische Phase umfassend vinylaromatische Monomere (M), die aus der Pyrolyse eines Polymers (P) erhalten wurden, und eine wässrige Phase umfassend mindestens ein Phosphatsalz in einer Menge, die zu einer Phosphatkonzentration (PO₄³⁻) von mindestens 0,01 mol/L, bevorzugt von mindestens 0,1 mol/L, mehr bevorzugt von 0,2 bis 2 mol/L, mehr bevorzugter von 0,4 bis 1 mol/L führt.

## Revendications

1. Procédé de production d'une huile de pyrolyse (PO) contenant des monomères vinylaromatiques (M) à partir d'un polymère (P) constitué d'unités répétitives desdits monomères (M), le procédé comprenant les étapes suivantes :
a. Pyrolyse du polymère (P) constitué d'unités répétitives de monomères vinylaromatiques (M) afin d'obtenir une vapeur (V) contenant lesdits monomères (M) ;
b. Condensation d'au moins une partie des monomères (M) en mettant la vapeur (V) en contact avec une solution aqueuse (A) ou de l'eau afin d'obtenir un mélange hétérogène composé d'une phase organique contenant des monomères vinylaromatiques (M) et d'une phase aqueuse ;
c. Exécution d'une séparation de phases entre la phase organique et la phase aqueuse ; et
d. Récupération de la phase organique contenant les monomères vinylaromatiques (M) afin d'obtenir l'huile de pyrolyse (PO) contenant les monomères vinylaromatiques (M),
à l'étape c. la phase aqueuse contenant au moins un sel de phosphate en quantité permettant d'obtenir une concentration en phosphate (PO₄³⁻) d'au moins 0,01 mol/L, de préférence d'au moins 0,1 mol/L, particulièrement de 0,2 à 2 mol/L, et plus particulièrement de 0,4 à 1 mol/L.

2. Procédé selon la revendication 1, **caractérisé en ce que** le procédé est continu.

3. Procédé selon la revendication 1 ou 2, **caractérisé en ce que** les monomères vinylaromatiques (M) comprennent du styrène, de préférence sont constitués de styrène.

4. Procédé selon l'une des revendications 1 à 3, **caractérisé en ce que** le polymère (P) provient de déchets de polymère, de préférence de déchets de polymère post-consommation.

5. Procédé selon l'une des revendications 1 à 4, **caractérisé en ce que** le procédé comprend en outre une étape supplémentaire, de préférence une étape de distillation, au cours de laquelle l'huile de pyrolyse (PO) ou certains de ses composants sont purifiés et/ou isolés.

6. Procédé selon l'une des revendications 1 à 5, **caractérisé en ce que**, à l'étape c., le rapport volumique entre la phase organique et la phase aqueuse est compris entre 95:5 et 5:95, de préférence entre 80:20 et 10:90, particulièrement entre 50:50 et 20:80, et plus particulièrement entre 40:60 et 25:75.

7. Procédé selon l'une des revendications 1 à 6, **caractérisé en ce que** l'au moins un sel de phosphate est introduit dans la solution aqueuse (A) utilisée à l'étape b. pour la condensation des monomères (M), ou est introduit dans le mélange hétérogène entre les étapes b. et c., ou à l'étape c. après obtention du mélange hétérogène à l'étape b., ou une combinaison de ces options.

8. Procédé selon l'une des revendications 1 à 7, **caractérisé en ce que** le sel de phosphate est choisi parmi les phosphates de métaux alcalins, les phosphates d'ammonium, leurs hydrates et leurs mélanges, de préférence parmi le groupe constitué de NaₙH₃₋ₙPO₄, KₙH₃₋ₙPO₄, (NH₄)ₙH₃₋ₙPO₄, et leurs hydrates, n étant un nombre entier compris entre 1 et 3, ainsi que leurs mélanges, particulièrement choisis parmi Na₃PO₄, K₃PO₄, (NH₄)₃PO₄, ainsi que leurs hydrates et leurs mélanges, plus particulièrement choisis parmi Na₃PO₄, K₃PO₄, leurs hydrates et leurs mélanges, et plus particulièrement K₃PO₄ et ses hydrates.

9. Procédé selon l'une des revendications 1 à 8, **caractérisé en ce que** la séparation de phases est effectuée à une température comprise entre 5 et 60 °C, de préférence entre 10 et 50 °C, et particulièrement entre 15 et 40 °C.

10. Procédé selon l'une des revendications 1 à 9, **caractérisé en ce que** l'étape a. comprend les phases suivantes :
a1. Fourniture d'une composition, de préférence des déchets de polymères, comprenant
30 à 100 % en poids, de préférence 50 à 99,9 % en poids, particulièrement 60 à 99 % en poids, et plus particulièrement 80 à 95 % en poids, par rapport au poids total de la composition, d'au moins un polymère (P) contenant des unités répétitives de monomères vinylaromatiques (M) ;
0 à 60 % en poids, de préférence 0 à 45 % en poids, particulièrement 0 à 37 % en poids, et plus particulièrement 0 à 18 % en poids, par rapport au poids total de la composition, de polyoléfines et/ou de polyalkyl(méth)acrylates ;
0 à 10 % en poids, de préférence 0 à 5 % en poids, particulièrement 0 à 3 % en poids, et plus particulièrement 0 à 2 % en poids, d'autres matériaux polymères ; et 0 à 40 % en poids, de préférence 0,1 à 35 % en poids, particulièrement 1 à 30 % en poids, et plus particulièrement 5 à 20 % en poids, par rapport au poids total de la composition, d'additifs et de composants non polymères ;
a2. Introduction continue de la composition obtenue à l'étape a1. dans la zone de réaction d'un réacteur de pyrolyse, la température de pyrolyse étant réglée à une valeur comprise entre 350 et 1 000 °C, de préférence entre 400 et 900 °C, particulièrement entre 500 et 800 °C, plus particulièrement entre 550 et 650 °C, et encore plus particulièrement entre 580 et 630 °C et le temps de séjour de la composition dans la zone de réaction étant réglé à une valeur comprise entre 0,1 seconde et 10 minutes, de préférence entre 0,2 seconde et 60 secondes, particulièrement entre 0,3 seconde et 10 secondes, afin de réaliser la pyrolyse du polymère (P) et des autres composants pyrolysables de la composition et d'obtenir une vapeur (V) contenant des monomères vinylaromatiques (M) ;
a3. Extraction continue de la vapeur (V) contenant des monomères vinylaromatiques (M) de la zone de réaction du réacteur de pyrolyse.

11. Système configuré pour mettre en œuvre le procédé selon l'une des revendications 1 à 10, comprenant un moyen permettant d'effectuer une pyrolyse d'un polymère (P) comprenant des unités répétitives de monomères vinylaromatiques (M), un moyen destiné à interrompre la réaction de pyrolyse à l'aide d'une solution aqueuse (A), un moyen destiné à effectuer une séparation de phases, un moyen destiné à recueillir la phase organique à partir du moyen destiné à effectuer la séparation de phases, et un moyen de dosage de sel configuré pour doser au moins un sel de phosphate dans le moyen destiné à effectuer la séparation de phases, et éventuellement un moyen de distillation.

12. Utilisation d'une solution aqueuse (A) contenant un sel de phosphate en quantité permettant d'obtenir une concentration en phosphate (PO₄³⁻) supérieure à 0 mol/L, de préférence d'au moins 0,01 mol/L, particulièrement d'au moins 0,1 mol/L, plus particulièrement entre 0,2 et 2 mol/L, et plus particulièrement entre 0,4 et 1 mol/L, afin de stopper une réaction de pyrolyse pour la récupération de monomères vinylaromatiques (M) à partir d'un polymère (P) comprenant des unités répétitives desdits monomères vinylaromatiques (M).

13. Procédé permettant d'accélérer la séparation de phases d'un mélange liquide hétérogène comprenant des monomères vinylaromatiques (M) obtenus par pyrolyse d'un polymère (P), et d'une solution aqueuse (A), le procédé comprenant l'étape consistant à ajuster la quantité d'ions phosphate dans la solution aqueuse (A) à une concentration d'au moins 0,01 mol/L, de préférence d'au moins 0,1 mol/L, particulièrement entre 0,2 et 2 mol/L, et plus particulièrement entre 0,4 mol/L et 1 mol/L.

14. Utilisation d'une solution aqueuse (A) contenant un sel de phosphate en quantité permettant d'obtenir une concentration en phosphate (PO₄³⁻) supérieure à 0 mol/L, de préférence d'au moins 0,01 mol/L, particulièrement d'au moins 0,1 mol/L, plus particulièrement entre 0,2 et 2 mol/L, et encore plus particulièrement entre 0,4 et 1 mol/L, pour le lavage d'une huile de pyrolyse (PO) contenant des monomères vinylaromatiques (M) obtenue par pyrolyse d'un polymère (P) comprenant des unités répétitives desdits monomères (M).

15. Mélange liquide hétérogène comprenant une phase organique constituée de monomères vinylaromatiques (M) issus de la pyrolyse d'un polymère (P), et d'une phase aqueuse comprenant au moins un sel de phosphate en quantité permettant d'obtenir une concentration en phosphate (PO₄³⁻) d'au moins 0,01 mol/L, de préférence d'au moins 0,1 mol/L, particulièrement entre 0,2 et 2 mol/L, et plus particulièrement entre 0,4 et 1 mol/L.
